# EUROPEAN PATENT SPECIFICATION

(11) **EP 0 651 645 B1**
(45) Date of publication and mention of the grant of the patent: **27.02.2002**
(21) Application number: 93918338.0
(22) Date of filing: 23.07.1993
(51) Int. Cl.: A61K 31/715

(54) **MEDICAMENT FOR INHIBITING NEUTROPHIL ELASTASE AND CATHEPSIN G**
ARZNEIMITTEL ZUR HEMMUNG DER NEUTROPHILEN ELASTASE UND CATHEPSIN G
MEDICAMENT PERMETTANT D'INHIBER LA CATHEPSINE G ET L'ELASTASE DE NEUTROPHILE

(30) Priority: 24.07.1992 US 919309
(43) Date of publication of application: 10.05.1995
(60) Divisional application: 01115601.5
(73) Proprietor: Kennedy, Thomas P., Richmond, VA 23294 (US)
(72) Inventor: Kennedy, Thomas P., Richmond, VA 23294 (US)
(74) Representative: Perry, Robert Edward
(86) International application number: US9306933
(87) International publication number: WO9402107

(56) References cited:
- EP-A- 0 208 623
- EP-A- 0 557 887
- WO-A-88/01280
- WO-A-92/01003
- BIOCHEMISTRY vol. 25, no. 18 , 1986 pages 5322 - 28 'Chemically modified heparins as inhibitors of heparan sulfate specific endo-beta-glucuronidase of metastatic melanoma cells'
- AM.REV.RESP.DIS. vol. 142 , 1990 pages 407 - 12 'Sulfated polysaccharides prevent human leukocyte elastase-induced acute lung injury and emphysema in hamsters' cited in the application
- BIOCHEMJ. vol. 252 , 1988 pages 515 - 19 'Inhibtion of leukocyte elastase by heparin and its derivatives'
- ISHAI MICHAELI ET AL: 'Importance of Size and Sulfatiuon of Heparin in Release of Basic Fibroblast Growth Factor from the Vascular Endothelium and ExtraCellular Matrix' BIOCHEMISTRY vol. 31, no. 7, 1992, pages 2080 - 2088
- BIOCHEMISTRY vol. 3, no. 28, 1992, pages 6498 - 6503
- WALDMAN ET AL.: 'Heparin as inhibitor of mammalian protein synthesis. II. Degree of sulfation. Ralated sulfated mucopolyysaaccharides' BIOCHIM. BIOPHYS. ACTA vol. 343, no. 2, pages 324 - 329, XP002056009
- CAN. J. CHEM. vol. 67, September 1989, pages 1449 - 1456

## Description

The present invention relates to a desulfated heparin and its use in medical treatment of mammals.

Activated neutrophils play an important role in a number of human and other mammalian diseases by releasing a number of oxidant chemicals and enzymes after migration into an affected organ. While oxidants, such as superoxide anion, hydrogen peroxide and hypochlorous acid are injurious by themselves, the major destructive elements produced by activated neutrophils are cationic proteases, the bulk of which consist of elastase and cathepsin G. When neutrophils release these proteases, tissue destruction occurs unless the proteases are neutralized by sufficient extracellular anti-proteinases such as α-1-anti-proteinase.

Individuals with an inherited deficiency of α-1-anti-proteinase suffer unimpeded proteolytic lung destruction over a lifetime, resulting ultimately in the development of pulmonary emphysema. Cigarette smoking causes the influx of activated leukocytes into the lung, with subsequent degranulation and release of proteases. Cigarette derived oxidants also inactivate α-1-anti-proteinase by oxidizing an important methionine near the active site. Elastase delivered to the alveolar lung unit as a result of the influx due to cigarette smoking, concurrent with oxidative inactivation of α-1-anti-proteinase activity, produces an imbalance of protease/anti-proteinase activity that is thought to be a major cause of human emphysema from cigarette smoking.

When the imbalance occurs within the airway, chronic airway inflammation is the result, and neutrophil derived elastase and cathepsin G are thought important in the pathogenesis of chronic bronchitis. If the imbalance occurs within the pulmonary vasculature, the resulting microvascular injury causes lung edema formation. In this fashion the influx of activated leukocytes and release of elastase and other neutrophil proteases are major causes of lung injury in the Adult Respiratory Distress Syndrome. Neutrophil derived elastase is also an important cause of proteolytic lung destruction in cystic fibrosis, a disease characterized by intense mucopurulent bronchitis and some of the highest levels of elastase activity measured in any human disease.

Also, diseases such as myocardial infarction and stroke, caused by sudden loss of organ blood flow, followed by blood flow restoration (ischemia-reperfusion injury) are characterized by magnification of tissue destruction during the reperfusion phase when activated leukocytes rapidly invade the already injured tissue. Neutrophil elastase delivered to ischemic reperfused organs has been demonstrated to play a pivotal role in the pathogenesis of reperfusion injury of the myocardium, bowel and other tissues. The role of cathepsin G in the processes above is not as well studied, but may be equally important, since there is twice as much cathepsin G present in the neutrophil as elastase.

Because elastase and cathepsin G are mediators of a variety of important human diseases, developing effective inhibitors of these enzymes is an active goal in experimental pharmacology. However, to date, no completely effective and safe inhibitor of both elastase and cathepsin G exists. An organic inhibitor of elastase has been developed (C.P. Sommerhoff, et al., European Journal of Pharmacology (1991) 193:153-158), but it failed to demonstrate activity against cathepsin G. Two biomolecules, α-1-anti-proteinase inhibitor and bronchial secretory inhibitor, are sensitive to inactivation by neutrophil oxidants and are not likely to be effective in biologic environments where neutrophil oxidants and proteases are present simultaneously (D. C. Flenley, Quarterly Journal of Medicine (1986) 61:901-909; C. Vogelmeier, et al., Journal of Clinical Investigation (1991) 87:482-488). An inhibitor is needed which is simultaneously effective against both elastase and cathepsin G but is impervious to either proteolytic or oxidative inactivation. The sulfated polysaccharides have each of those desirable qualities.

It has been previously reported that heparin and other sulfated polysaccharides are potent non-competitive inhibitors of elastase and cathepsin G from human polymorphonuclear leukocytes (N. V. Rao, et al., A. M. Rev. Respir. Dis. (1990) 142:407-412; A. Baici, et al., Biochem. Pharmacol. (1980) 29:1723-1727; A. Baichi, et al., Biochem. Pharmacol. (1981) 30:703-708; K. Marossy, Biochim. Biophys, Acta, (1981) 659:351-361; A. Bacici, et al., Chem-Biol. Interactions (1984) 51:1-11; A. Lutini, et al., Biochem. Int. (1985) 10:221-232; F. Redini, et al., Biochem. J. (1988) 252:515-519; and F. Redini, et al., Biochem. Pharmacol. (1988) 37:4257-4261.) It is believed that the basis for inhibition is the formation of electrostatic bonds between the negatively charged sulfate groups of the polysaccharide and the positively charged guanidinium groups of the arginine residues located at the surface of those highly basic enzymes such as elastase or cathepsin G. The interaction does not influence the active center of the enzyme but causes an indirect loss of elastolytic activity.

Of all the sulfated polysaccharides, heparin has the longest and safest history of use in man. From a toxicologic consideration, heparin is the most desirable inhibitor of elastase and cathepsin G but it is an anti-coagulant even when delivered selectively to the lung by aerosol. It is believed that heparin acts as an anti-coagulant because of a repeated sequence of saccharides which binds specifically to the plasma protein anti-thrombin III, dramatically accelerating the rate at which anti-thrombin inhibits the procoagulant effect of thrombin on the cascade of blood coagulation. only a portion of commercial heparin binds to anti-thrombin III, and passage of heparin over an affinity column of anti-thrombin III-sepharose removes the anti-coagulant fraction leaving an incompletely sulfated fraction devoid of anti-coagulant activity. However, utilizing this process to rid heparin of its anti-coagulant activity is too inefficient to be undertaken on a commercially practical scale.

In the prior art, it has been noted that the activity of a polysaccharide as an inhibitor of human polymorphonuclear leucocyte elastase (HLE) and cathepsin G is directly dependent upon the presence of intact sulfate groups. Dextran sulfate is a potent inhibitor of elastase, but non-sulfated dextran is not. Furthermore, the available literature suggests that even partial desulfation of polysaccharides eliminates inhibitory activity toward HLE and cathepsin G while chemical oversulfation enhances inhibitory activity. The importance of sulfate groups was studied using fragments of heparin obtained by chemical depolymerization with HNO₂ followed by gel filtration (F. Redini, et al., Biochem. J., (1988) 252:515-519). Unmodified heparin fragments obtained by this latter process were potent inhibitors of elastase but retained their strong anticoagulant power. Increasing the degree of sulfation by chemical 0-sulfation of the fragments markedly increased their potency as elastase inhibitors but did not materially alter the anticoagulant activity of the fragments. On the other hand, N-desulfation followed by N-acetylation (to cover the remaining positive charge and reduce the anticoagulant activity of the fragments) completely eliminated inhibitory activity toward human leukocyte elastase and cathepsin G. Chemical over-O-sulfation of the N-desulfated fragments not only restored inhibitory activity but gave the fragments higher inhibitory potential compared to their original counterparts with a similar degree of sulfation but containing N-sulfate groups. It has been suggested that not only was the degree of sulfation important to inhibitory activity, but that the presence of O-sulfates were more important than the presence of N-sulfates. However, none of these highly effective, modified heparins were suitable for use in mammals due to their potent continuing anticoagulant activity.

Several chemical methods exist for inactivating heparin as an anti-coagulant. Most are based on techniques of chemical desulfation, since it is well established that degree of sulfation is an important determinant of anticoagulant activity.

N-desulfation by treatment of the pyridinium heparin salt with dimethylsulfoxide (DMSO) in five percent methanol for 1.5 hours at 50°C and total desulfation by similar treatment in DMSO in 10% methanol for 18 hours at 100°C are commonly used chemical modifications to remove anti-coagulant activity from heparin. Another method to remove anti-coagulant activity from heparin is acid hydrolysis at 55-60°C for 72 hours to produce partial N-desulfation. However, removal of all sulfates or even a partial desulfation by removal of N-sulfates inactivates heparin and other sulfated polysaccharides as inhibitors of human elastase and cathepsin G. Thus, the prior art teaches that currently utilized desulfation methods which remove anti-coagulant activity of heparin also destroy its ability to inhibit cationic leukocyte proteases such as elastase and cathepsin G.

Thus, the prior art demonstrates that oversulfation leads to increased activity against elastase and cathepsin G with continuing anticoagulant activity while desulfation leads to decreased anticoagulant activity with greatly diminished activity against elastase and cathepsin G. In contrast to what would be predicted by the prior art, the present inventors have discovered that selective 2-O-desulfation of α-L-iduronic acid-2-sulfate eliminates the anticoagulant activity of heparin without destroying the activity of the modified heparin as an inhibitor of elastase and cathepsin G.

It is an object of the present invention to provide a medicament for inhibiting elastase and cathepsin G in mammals.

It is another object of the present invention to provide such inhibition in a therapeutic agent which substantially does not induce anti-coagulant activity.

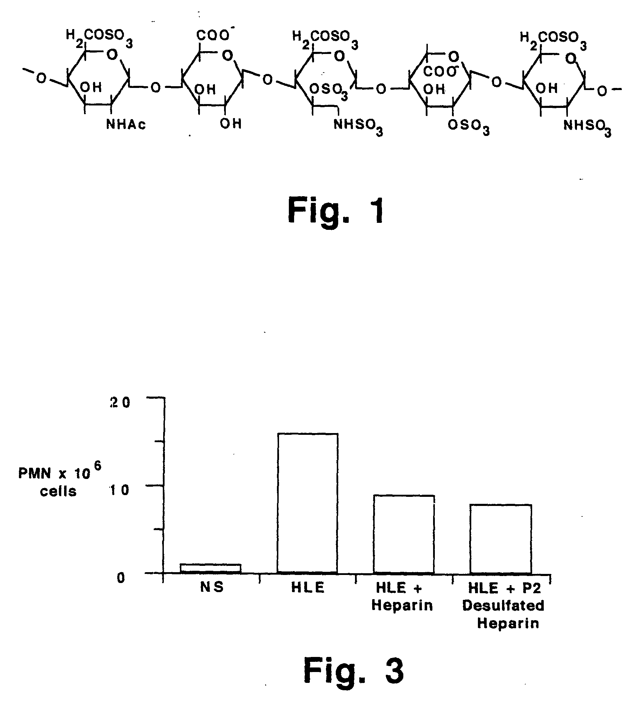

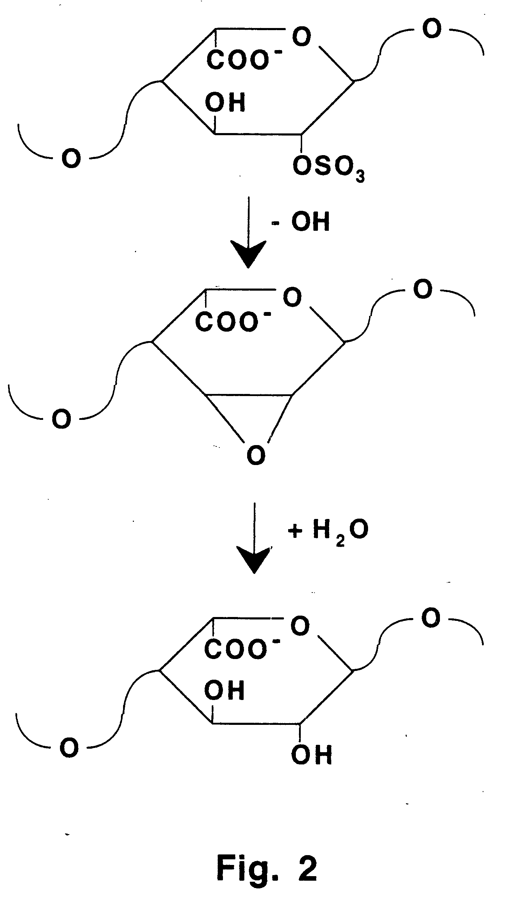

The removal of the 2-O-sulfate from α-L-iduronic acid in the pentasaccharide binding sequence of heparin, shown in Figure 1, occurs due to the chemistry of sulfated carbohydrates in alkaline conditions which was first elucidated by E.G.V. Percival, Quarterly Rev. (1949) 3:369-384, and reviewed in J.P.R. Turvey, Adv. Carbohydrate Chem. (1965) 20:183-218. A sulfate group on a secondary hydroxyl group is susceptible to alkaline hydrolysis if there is an adjacent, *trans,* free hydroxyl group available for forming an epoxide intermediate as the sulfate is eliminated. This proposed reaction scheme for 2-O-desulfation is shown in Figure 2. For heparin, this reaction has been recently reported to occur with alkaline hydrolysis during lyophilisation. See R. Rej *et al*, Thromb. Haemostas. (1989) 61:540, and M. Jaseja *et al*, Can. J. Chem. (1989) 67:1449-1456.

According to the present invention, a non-anticoagulant desulfated heparin is defined in claim 1 (and may be described herein as "2-O-desulfated heparin"). This product is useful in therapy. In particular, the desulfated heparin substantially inhibits elastase and cathepsin G without inducing anti-coagulant activity.

A medicament comprising the desulfated heparin of this invention is preferably administered by aerosolisation or by intravenous injection. The effective ratio of 2-O-desulfated heparin to elastase is chosen to be greater than 0.2 and less than 2.0; the effective ratio of 2-O-desulfated heparin to cathepsin G is chosen to be greater than 0.4 and less than 2.0. Preferably, the medicament includes a physiologically-acceptable carrier which may be selected from physiologically-buffered saline, normal saline, and distilled water.

The following Examples illustrate the invention.

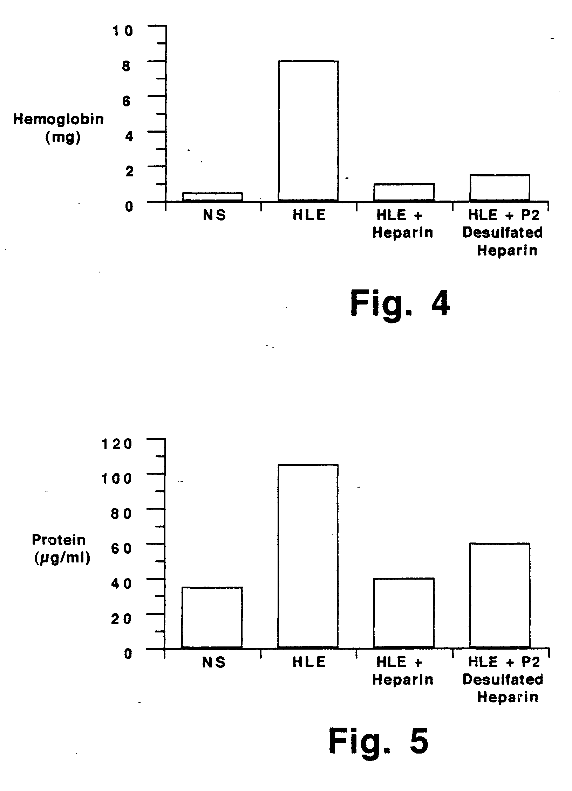

Figure 3 shows a graph of the number of polymorphonuclear leukocyte (PMN) cells in the bronchoalveolar lavage fluid 24 hours after the administration of saline (control), human leukocyte , elastase (HLE), HLE plus heparin, and HLE plus desulfated heparin;
Figure 4 shows a graph of the hemoglobin content measured in the bronchoalveolar lavage fluid 24 hours after the administration of saline (control), HLE, HLE plus heparin, and HLE plus desulfated heparin; and
Figure 5 shows a graph of the concentration of protein in the bronchoalveolar lavage fluid 24 hours after administration of saline (control), HLE, HLE plus heparin, and HLE plus desulfated heparin.

### EXAMPLE I

### Partial N-Desulfation of Heparin

Porcine intestinal mucosal heparin (Scientific Protein Laboratories, Waunakee, WI) was converted to heparinic acid by passing a 4% aqueous solution over a cation exchange resin column, Dowex 50W x 8X (H⁺). The solution of heparinic acid was then kept at 55°C for 72 hours in a standard chemical reflux apparatus to remove approximately 70% of N-sulfates, as reported by L. Levy et al., Proc. Soc. Exp. Biol. Med. (1962) 109:901-905 and E. Sache et al., Thromb, Res, (1989) 55:247-258. The N-desulfated heparin was recovered by passing the solution over IR-400 resin (OH⁻) to remove excess SO₄⁻ followed by adjustment of pH to 7.0 and lyophilization.

### EXAMPLE II

### Interaction of Partially N-Desulfated Heparin with HLE

The inhibition of HLE by partially N-desulfated heparin of Example I was measured by incubating a constant amount of HLE (100 pmol) with increasing amounts of partially N-desulfated heparin (10-50 pmol, I:E ratio 0.1-0.5) for 30 minutes at 25°C in 500 microliters of Hepes buffer (0.125 M, 0.125% Triton-X-100, pH 7.5) diluted to the final volume of 900 microliters. The remaining enzyme activity was measured by adding 100 microliters of 3mM N-Suc-Ala-Ala-Val-NA (Sigma Chemicals, St. Louis, MO, made in dimethylsulfoxide [DMSO]) and reading the absorbance of the proteolytically released chromogen 4-Nitroaniline at 405 nm. The percentage of inhibition was calculated based on enzyme activity without inhibitor.

The results of the interaction of partially N-desulfated heparin and heparin with HLE are shown in Table I.

**TABLE I**

| PERCENT INHIBITION OF HLE | | |
|---|---|---|
| Substrate Inhibito^{a} Ratio I:E | H | NDH |
| 0.1:1 | 53 | 29 |
| 0.2:1 | 79 | 37 |
| 0.3:1 | 88 | 37 |
| 0.4:1 | 90 | 49 |
| 0.5:1 | 91 | 50 |

| | | |
|---|---|---|
| ^{a}H = Heparin; NDH = Heparin partially N-desulfated according to Example I | | |

As shown in Table I, heparin significantly inhibited human leukocyte elastase (HLE) at I:E ratios of greater than 0.2. In contrast, heparin partially N-desulfated according to Example I had greatly reduced ability to inhibit HLE, even at I:E ratios of 0.5. Thus, the activity of heparin as an inhibitor of HLE was substantially diminished by even partial desulfation of the polysaccharide by this treatment commonly used to eliminate the anticoagulant effect of heparin. These results were consistent with previously discussed prior art suggesting that desulfation destroys the activity of heparin as an inhibitcr of HLE and cathepsin G.

### EXAMPLE III

### 2-O-Desulfation of Heparin:

An aqueous solution of 0.4% porcine intestinal mucosal heparin (Scientific Protein Laboratories, Waunakee, WI) (4 mg/ml) was adjusted to pH 13.0 with 0.1 N NaOH, frozen, then lyophilized in 40 ml aliquots. After the product was redissolved in water and passed over an Amberlite IR-120 plus (H+) cation exchange resin to remove excess sodium hydroxide, final pH was adjusted to 7.0, and the solution was passed through a 0.2 micron Millipore filter by vacuum filtration to ensure bacterial sterility prior to final re-lyophilization to dryness.

For commercial preparation of larger amounts of the 2-0-desulfated heparin, aqueous solutions (up to 5% weight/volume) of porcine intestinal mucosal heparin (Scientific Protein Labora-tories, Waunakee, WI), sodium salt, are adjusted to pH 13.0 by addition of NaOH, followed by lyophilization. To remove excess NaOH, the dry product is redissolved in aqueous solution, and heparin is precipitated by the addition of three volumes of cold (4°C) ethanol. The mixture is stored at 4°C for 12 hours to allow complete precipitate formation, then centrifuged. After drying to evaporate excess ethanol, the precipitated heparin is redissolved in aqueous solution, pH 7.0, filtered through a 0.2 micron Millipore filter, and relyophilized to dryness.

### EXAMPLE IV

### Effect of 2-O-Desulfated Heparin on Blood Coagulation:

The anti-coagulant potential of the desulfated heparin from Example III was studied by determining its effect on the activated partial thromboplastin time (APPT) in vitro. The test was performed in the usual fashion used to monitor the anti-coagulant effect of heparin clinically in patients. The test used 0.1 and 1.0 mg/ml heparin or heparin, 2-O-desulfated according to Example III, added to human test serum in vitro.

**TABLE II**

| | Control | Heparin | 2-O-Desulfated Heparin | | |
|---|---|---|---|---|---|
| Conc. (mg/ml) | 0 | 1.0 | 0.1 | 1.0 | 0.1 |
| Time to Clot Formation (sec) | 35-45 | >150 | 80 | 42 | 38 |

The 2-0-desulfated heparin from Example III was also studied to determine whether plasma dilutions of 0.1 mg/ml heparin or heparin, desulfated according to Example III, inhibited factor Xa, prolonging test time in an assay for Xa activity utilizing plasma treated with Russell viper venom.

**TABLE III**

| Dilution | Anti-factor Xa Activity | | |
|---|---|---|---|
| | Control Plasma | Heparin | 2-0-Desulfated Heparin |
| 1:2 | | > 8 min. | 42 sec. |
| 1:10 | | > 7 min. | 33 sec. |
| 1:100 | | 42 sec. | 32 sec. |
| 1:1000 | | 32 sec. | 32 sec. |
| 0 | 35 sec. | | |

In contrast to heparin, the heparin desulfated according to Example III showed little ability to prolong the APTT and little antifactor Xa activity.

Thus, the 2-0-desulfated heparin showed a much reduced anti-coagulant activity when compared to non-desulfated heparin.

### EXAMPLE V

### Interaction of 2-O-Desulfated Heparin with HLE and Cathepsin G:

The inhibition of HLE by 2-0-desulfated heparin of Example III was measured by incubating a constant amount of HLE (100 pmol) with increasing amounts of 2-0-desulfated heparin (10-60 pmol, I/E ratio 0.1-0.6) for 30 minutes at 25°C in 500 microliters of Hepes buffer (0.125 M, 0.125% Triton-X-100, pH 7.5) diluted to the final volume of 900 microliters. The remaining enzyme activity was measured by adding 100 microliters of 3 mM N-Suc-Ala-Ala-Val-NA (Sigma Chemicals, St. Louis, MO, made in dimethylsulfoxide [DMSO]) and reading the absorbance of the proteolytically released chromogen 4-Nitroaniline at 405 nm. The percentage of inhibition was calculated based on enzyme activity without inhibitor.

The inhibition of cathepsin G by 2-0-desulfated heparin of Example III was measured as described above, except that the substrate was 3 mM Suc-Ala-Ala-Pro-Phe-pNA (Sigma Chemical, in DMSO).

Also, bovine ligament elastin was prepared according to B. C. Starcher, Anal. Biochem. (1976) 74:441-447. The elastin was assessed for purity by amino acid analysis. Its degradation was assayed using elastin radiolabeled with ³H-NaBH₄, following the methods described in P. J. Stone, et al., Methods Enzymol. (1982) 82:588-605. The tritiated powdered elastin was homogenized and washed in phosphate buffered saline (PBS), pH 7.4 just before use to remove unincorporated radioactivity. A constant amount of human leukocyte elastase (HLE) (67 pmol) was preincubated with increasing amounts of 2-0-desulfated heparin (6.7-134 pmol) at 37°C for 30 minutes in a final volume of 1.0 ml Hepes buffer (0.125 M, 0.125% Triton-X-100, pH 7.5). An aliquot of 900 microliters of each reaction mixture was incubated with 5 mg ³H-elastin and 100 microliters of 0.9% saline at 37°C. Solubilized peptides were separated from the elastin suspension by filtration through medium-porosity filter paper. The rate of degradation was determined by quantifying the solubilized ³H peptides.

The results of the interactions of 2-O-desulfated heparin and heparin with HLE and cathepsin G are shown in Table IV.

**TABLE IV**

| PERCENT INHIBITION | | | | | | |
|---|---|---|---|---|---|---|
| | HLE | | | | Cathepsin G | |
| Substrate^{a} | A | | B | | C | |
| Inhibitor^{b} | H | ODH | H | ODH | H | ODH |
| Ratio I:E | | | | | | |
| 0.1:1 | 23 | 20 | 72 | 73 | 31 | 14 |
| 0.2:1 | 81 | 74 | 95 | 77 | 49 | 54 |
| 0.3:1 | 88 | 84 | - | - | - | - |
| 0.4:1 | 87 | 85 | 92 | 72 | 67 | 56 |
| 0.5:1 | 87 | 84 | 94 | 85 | - | - |
| 0.6:1 | 99 | 100 | - | - | - | - |
| 0.8:1 | - | - | - | - | 78 | 69 |
| 1.0:1 | - | - | 97 | 85 | 76 | 69 |
| 2.0:1 | - | - | 97 | 84 | 76 | 67 |

| | | | | | | |
|---|---|---|---|---|---|---|
| ^{a} A = N-Suc-Ala-Ala-Val-NA; B = ³H-Elastin; C = Suc-Ala-Ala-Pro-Phe-pNA. | | | | | | |
| ^{b} H = Heparin; ODH = 2-0-Desulfated Heparin according to Example III | | | | | | |

As is shown in Table IV, ratios of inhibitor to substrate (I:E) of greater than 0.2 produces significant inhibition of human leukocyte elastase (HLE), I:E ratios of greater than 0.4 also provides significant inhibition of cathepsin G by both heparin and heparin 2-0-desulfated according to Example III. Throughout the range of ratios, there is very little difference between the effective inhibition of heparin and the heparin 2-0-desulfated according to Example III. Thus, the 2-0-desulfated heparin provides substantially the same inhibition as does the non-desulfated heparin. In addition, the 2-0-desulfated heparin showed little anticoagulant activity. This is in contrast to the unmodified heparin which is a very active anticoagulation agent.

### EXAMPLE VI

### In Vivo Studies:

The ability of desulfated heparin to prevent human leukocyte elastase (HLE)-mediated lung injury was assessed in female golden Syrian hamsters (Harlan Industries, Indianapolis, Indiana) weighing 90 to 110 g. Phenobarbital-anesthesitized hamsters were injected intratracheally with 0.25 ml sterile 0.9% saline (NS), 0.25 ml NS containing HLE (100 µg), or 0.25 ml NS containing 500 µg of heparin (Sigma) or 2-0-desulfated heparin according to Example III followed by 0.25 ml NS with HLE. Animals were killed by exsanguination 24 hours after treatment. The throat was opened and lungs dissected en bloc. The trachea was cannulated with polyethylene tubing and lavaged with five sequential aliquots of 3 ml NS. Lavage fluid was centrifuged at 200 Xg for 10 minutes. The resulting cell pellet was re-suspended in 1 ml Hank's balanced salt solution (HBSS) for performing cell count and differentials. The supernatant was assayed for protein and hemoglobin, as indices of acute injury. The results are:

**TABLE V**

| | PMN^{a} (X10⁶ Cells) | Total Hemoglobin (mg) | Protein (mg/ml) |
|---|---|---|---|
| Injected Solution^{b} | | | |
| Control | 0.95 | 0.396 | 35.08 |
| 0.5 ml NS | (0.443) | (0.215) | (0.111) |
| HLE | 16.3 | 8.15 | 100.69 |
| (100 µg) | (0.744) | (0.53) | (0.98) |
| HLE + H | 10.83 | 0.867 | 41.06 |
| (100 µg + 500 µg) | (0.452) | (0.439) | (0.114) |
| HLE + ODH | 9.83 | 1.5 | 65.80 |
| (100 µg + 500 µg) | (0.86) | (0.23) | (0.659) |

| | | | |
|---|---|---|---|
| ^{a} PMN = polymorphonuclear leukocyte | | | |
| ^{b} HLE = human leukocyte elastase; H = heparin; ODH = 2-0-desulfated heparin according to Example II | | | |

Both heparin and 2-0-desulfated heparin, according to Example III, were potent inhibitors of elastase induced injury in vivo.

The 2-O-desulfated heparin from Example III has been tested for toxicity. Other sulfated polysaccharide inhibitors of elastase and cathepsin G, such as dextran sulfate, produced hemorrhage into lung air sacs (alveolar hemorrhage) when injected into rats intratracheally in doses as low as 0.5 mg/kg. The 2-O-desulfated heparin from Example III produced no alveolar hemorrhage in rats even in intratracheal doses of 10 mg/kg.

The alkaline hydrolyzed heparin produced according to Example III can be used to treat respiratory diseases such as emphysema, chronic bronchitis, Adult Respiratory Distress Syndrome (ARDS) and cystic fibrosis by administration to the respiratory tree directly as an aerosol. A dose of from about 10 to about 100 mg of 2-0-desulfated heparin dissolved in 3 ml of sterile 0.9% saline is aerosolized into the lung about every 6 hours (or about 4 times daily) using any common clinically available aerosol device (such as a Devilbiss or Acorn Nebulizer) attached to a positive pressure source (either compressor or compressed air or oxygen) to generate aerosols of particles less than 10 microns mass median diameter.

The lower doses would be effective for diseases such as chronic bronchitis, whereas the higher doses would be needed for cystic fibrosis, in which the levels of elastase in respiratory secretions are much higher.

For treatment of diseases of ischemia-reperfusion such as myocardial infarction and stroke, the 2-0- desulfated heparin would be administered intravenously (i.v.) by continuous infusion. After a bolus loading dose of 0.5 mg/kg intravenously, 2.5-5.0 mg/kg of 2-0-desulfated heparin is mixed with 250-500 ml of 5% dextrose, 0.45% NaCl or 0.9% NaCl and infused continuously over 24 hours to maintain a constant blood level of drug within the vascular system.

## Claims

1. A non-anticoagulant desulfated heparin, obtainable by adjusting an aqueosus solution (up to 5% weight/volume) of porine intestinal mucosal heparin, sodium salt, to pH 13.0 by addition of NaOH, followed by lyophilisation, removal of excess NaOH by redissolving the dry product in aqueous solution, precipitation of heparin by the addition of 3 volumes of cold (4°C) ethanol, storage of the mixture at 4°C for 12 hours to allow complete precipitation, centrifugation, drying to remove excess ethanol, redissolving the precipitated heparin in aqueous solution, pH 7.0, filtration through a 0.2 µm filter, and relyophilisation to dryness, for use in therapy.

2. A desulfated heparin of claim 1, for use in the treatment of emphysema, chronic bronchitis, adult respiratory distress syndrome or cystic fibrosis.

3. A desulfated heparin of claim 1, for use in the treatment of a disease of ischemia-reperfusion.

4. A desulfated heparin of claim 1, for use in the treatment of myocardial infarction or stroke.

5. A medicament comprising a desulfated heparin of any preceding claim. 5

6. A medicament of claim 5, adapted to be administered by aerosolisation.

7. A medicament of claim 5, adapted to be administered by intravenous injection.

8. A medicament of any of claims 5 to 7, comprising a carrier selected from physiologically-buffered saline, normal saline and distilled water.

9. Use of a desulfated heparin as defined in claim 1, for the manufacture of a medicament for the treatment of emphysema, chronic bronchitis, adult respiratory distress syndrome, cystic fibrosis or a disease of ischemia-reperfusion.

10. The use of claim 9, wherein the medicament is for use in the treatment of emphysema, chronic bronchitis, adult respiratory distress syndrome or cystic fibrosis.

11. The use of claim 9, wherein the medicament is for use in the treatment of a disease of ischemia-reperfusion.

12. The use of claim 11, wherein the disease is myocardial infarction or stroke.

13. The use of any of claims 9 to 12, wherein the medicament is adapted to be administered by aerosolisation.

14. The use of any of claims 9 to 12, wherein the medicament is adapted to be administered by intravenous injection.

15. The use of any of claims 9 to 14, wherein the medicament comprises a carrier selected from physiologically-buffered saline, normal saline and distilled water.

## Patentansprüche

1. Nicht gerinnungshemmendes desulfatiertes Heparin, erhältlich durch Einstellung einer wäßrigen Lösung (bis zu 5 % (Gew./Vol.)) von Schweinedarmschleimhaut-Heparin, Natriumsalz, auf pH 13,0 durch Zugabe von NaOH, gefolgt von Lyophilisieren, Entfernen von überschüssigem NaOH durch erneutes Auflösen des trockenen Produkts in wäßriger Lösung, Ausfällung von Heparin durch Zugabe von 3 Volumina an kaltem (4°C) Ethanol, Lagerung des Gemisches bei 4°C für 12 Stunden, um eine vollständige Ausfällung zu erlauben, Zentrifugation, Trocknen zur Entfernung von überschüssigem Ethanol, erneutem Auflösen des gefällten Heparins in wäßriger Lösung, pH 7,0, Filtration durch einen 0,2 µm-Filter und erneutes Lyophilisieren zur Trockene; zur therapeutischen Verwendung.

2. Desulfatiertes Heparin nach Anspruch 1 zur Verwendung bei der Behandlung von Emphysem, chronischer Bronchitis, Atemdepression ("adult respiratory distress syndrome") oder zystischer Fibrose.

3. Desulfatiertes Heparin nach Anspruch 1 zur Verwendung bei der Behandlung einer Erkrankung von Ischämie-Reperfusion.

4. Desulfatiertes Heparin nach Anspruch 1 zur Verwendung bei der Behandlung von Myokardinfarkt oder Schlaganfall.

5. Medikament, umfassend desulfatiertes Heparin nach einem der vorstehenden Ansprüche.

6. Medikament nach Anspruch 5, angepaßt für die Verabreichung durch Aerosolisation.

7. Medikament nach Anspruch 5, angepaßt für die Verabreichung durch intravenöse Injektion.

8. Medikament nach einem der Ansprüche 5 bis 7, umfassend einen Träger, der unter physiologisch gepufferter Kochsalzlösung, normaler Kochsalzlösung und destilliertem Wasser ausgewählt ist.

9. Verwendung von desulfatiertem Heparin gemäß der Definition in Anspruch 1 zur Herstellung eines Medikamentes für die Behandlung von Emphysem, chronischer Bronchitis, Atemdepression ("adult respiratory distress syndrome"), zystischer Fibrose oder einer Erkrankung von Ischämie-Reperfusion.

10. Verwendung nach Anspruch 9, wobei das Medikament für die Verwendung bei der Behandlung von Emphysem, chronischer Bronchitis, Atemdepression ("adult respiratory distress syndrome") oder zystischer Fibrose vorgesehen ist.

11. Verwendung nach Anspruch 9, wobei das Medikament für die Verwendung bei der Behandlung einer Erkrankung von Ischämie-Reperfusion vorgesehen ist.

12. Verwendung nach Anspruch 11, wobei es sich bei der Erkrankung um Myokardinfarkt oder Schlaganfall handelt.

13. Verwendung nach einem der Ansprüche 9 bis 12, wobei das Medikament für die Verabreichung durch Aerosolisation angepaßt ist.

14. Verwendung nach einem der Ansprüche 9 bis 12, wobei das Medikament für die Verabreichung durch intravenöse Injektion angepaßt ist.

15. Verwendung nach einem der Ansprüche 9 bis 14, wobei das Medikament einen Träger umfaßt, der unter physiologisch gepufferter Kochsalzlösung, normaler Kochsalzlösung und destilliertem Wasser ausgewählt ist.

## Revendications

1. Héparine désulfatée non anticoagulante, pouvant être obtenue par ajustement à pH 13,0, par addition de NaOH, une solution aqueuse (jusqu'à 5 % en poids/volume) d'un sel de sodium d'héparine de muqueuse intestinale de porc, ensuite lyophilisation, élimination du NaOH en excès par redissolution du produit sec dans une solution aqueuse, précipitation de l'héparine par addition de 3 volumes d'éthanol froid (4° C), conservation du mélange à 4° C pendant douze heures pour permettre une précipitation complète, centrifugation, séchage pour éliminer l'éthanol en excès, redissolution de l'héparine précipitée dans une solution aqueuse à pH 7,0, filtration à travers un filtre de 0,2 µm et re-lyophilisation jusqu'à siccité, pour une utilisation en thérapie.

2. Héparine désulfatée selon la revendication 1, destinée à être utilisée dans le traitement de l'emphysème, de la bronchite chronique, du syndrome de détresse respiratoire de l'adulte ou de la fibrose cystique.

3. Héparine désulfatée selon la revendication 1, destinée à être utilisée dans le traitement d'une affection d'ischémie - reperfusion.

4. Héparine désulfatée selon la revendication 1, destinée à être utilisée dans le traitement de l'infarctus du myocarde ou de la crise cardiaque.

5. Médicament comprenant une héparine désulfatée selon l'une quelconque des revendications précédentes.

6. Médicament selon la revendication 5, adapté pour être administré par aérosol.

7. Médicament selon la revendication 5, adapté pour être administré par injection intraveineuse.

8. Médicament selon l'une quelconque des revendications 5 à 7, comprenant un porteur sélectionné parmi une solution saline tamponnée physiologiquement, une solution saline normale et l'eau distillée.

9. Utilisation d'une héparine désulfatée selon la revendication 1 pour la fabrication d'un médicament en vue du traitement de l'emphysème, de la bronchite chronique, du syndrome de détresse respiratoire chez l'adulte, de la fibrose cystique ou d'une affection d'ischémie - reperfusion.

10. Utilisation selon la revendication 9, dans laquelle le médicament est destiné à être utilisé dans le traitement de l'emphysème, de la bronchite chronique, du syndrome de détresse respiratoire chez l'adulte ou de la fibrose cystique.

11. Utilisation selon la revendication 9, dans laquelle le médicament a destiné à être utilisé dans le traitement d'une affection résultant d'une ischémie - reperfusion.

12. Utilisation selon la revendication 11, dans laquelle la maladie est l'infarctus du myocarde ou la crise cardiaque.

13. Utilisation selon l'une quelconque des revendications 9 à 12, dans laquelle le médicament est adapté pour être administré par aérosol.

14. Utilisation selon l'une quelconque des revendications 9 à 12, dans laquelle le médicament est adapté pour être administré par injection intraveineuse.

15. Utilisation selon l'une quelconque des revendications 9 à 14, dans laquelle le médicament comprend un porteur sélectionné parmi une solution saline physiologiquement tamponnée, une solution saline normale et l'eau distillée.
